# EUROPEAN PATENT SPECIFICATION

(11) **EP 1 892 010 B1**
(45) Date of publication and mention of the grant of the patent: **06.10.2010**
(21) Application number: 06119526.9
(22) Date of filing: 25.08.2006
(51) Int. Cl.: A61M 25/10, A61J 15/00, A61B 5/00

(54) **Enteral feeding catheter and apparatus for determining the intraabdominal pressure of a patient**
Katheter für die enterale Ernährung und Vorrichtung zur Determinierung des intraabdominalen Druckes
Cathéter pour l'alimentation entérale et dispositif pour déterminer la pression intraabdominale

(43) Date of publication of application: 27.02.2008
(73) Proprietor: Pulsion Medical Systems AG, 81829 München (DE)
(72) Inventor: Dr. Malbrain, Manu, 3360, Lovenjoel (BE); Dr. Goedje, Oliver, 82031, Grünwald (DE); Herz, Robert, 83101, Rohrdorf (DE); Bohn, Matthias, 81477, München (DE)
(74) Representative: Ettmayr, Andreas

(56) References cited:
- WO-A-86/03957
- US-A- 3 046 988
- US-A- 5 460 603
- US-A- 5 599 301
- US-A- 5 718 685
- US-A1- 2001 053 920
- US-A1- 2002 013 537

## Description

The present invention relates to enteral feeding catheters for channelling flowable nutrient into a patient's digestive tract, such as into patient's stomach, duodenum or jejunum, which are adapted to be advanced through patient's esophagus. More particularly, the present invention relates to an enteral feeding catheter which has a proximal end, a distal end, and a feeding lumen having an opening at the proximal end adapted to be connected to a supply for receiving nutrient and channelling the nutrient from the proximal end to the distal end, wherein the distal end is provided with radial outlets for the exit of the nutrient.

Feeding catheters of this type are used for instance in feeding of a patient who is unable to swallow nutrients in the natural manner or in postoperative or critical care.

It has been known from US 5,462,528 to equip enteral feeding catheters with an inflatable balloon near the distal end in order to seal the contents of the stomach off from the esophagus. In order to ensure keeping such an enteral feeding catheter in its desired position, US 5,718,685 suggests using an additional inflatable balloon, which is positioned in the esophagus in a short distance from the sealing balloon. A pressure gauge is used to control inflation of the balloons, in particular to determine when the balloons have reached a state of inflation sufficient to perform their function of holding, sealing and stabilizing.

Equipping an enteral feeding catheter with inflatable balloons for a different purpose has been disclosed in US 3;055;371. Therein, inflatable balloons are used to tamponade for hemorrhage and the like from esophago-gastric varices.

Especially in critical care it is important to monitor the intra-abdominal pressure in order to avoid intra-abdominal hypertension or abdominal compartment syndrom, which may impede blood circulation and perfusion in the abdominal region and may be hazardous to the patient.

In the US patent application US 3,046,988 A an esophageal nasogastric tube is described having an gastric balloon whereby hemorrhaging in the fundus of the stomach and adjacent areas is effectively controlled. US 3,046,988 A does not disclose an enteral feeding catheter comprising a reflux filter arranged between a distal balloon and a proximal end of said measurement lumen.

DE 35 00 822 A1 discloses a device for measuring the pressure in the human or animal body. The device comprises a balloon which is attached at the distal end of a multi-luminal catheter and which is only partially filled with a gaseous medium. The pressure in the balloon is transferred over a lumen of the catheter to a pressure gauge. DE 35 00 822 A1 does not disclose an enteral feeding catheter.

It is an object of the present invention to provide a device which allows reliable continuous determination of intra-abdominal pressure of a patient, who is fed by an enteral feeding catheter and which keeps the additional burden related to the pressure measurement for the patient as low as possible. It is further an object of the invention to protect sensitive measurement equipment used therein from aggressive gastric juices and in particular to avoid electric contact between electronic components and stomach contents.

Under one aspect of the present invention, the above objects are achieved by an enteral feeding catheter according to claim 1. Advantageous embodiments of the present invention can be configured according to any of claims 2-14.

Under another aspect of the present invention, the above objects are achieved by an apparatus according to claim 15. Advantageous embodiments of the present invention can be configured according to any of claims 16-24.

According to the present invention, an enflatable gastric balloon - which will be named "distal balloon" in the following just for the sake to give it a name and to distinguish it from other balloons (described later) which are situated proximally with respect to the distal balloon - connected to a pressure gauge via a measurement lumen is usable to determine intra-abdominal pressure. In this context, "distal balloon" must not be understood in the sense that the distal balloon is situated at the very distal end of the catheter body. In order to protect the pressure gauge and to avoid electric connection between electronic components and the patient in case of rupture of the distal balloon, a filter is disposed in the gas path between the distal balloon and the pressure gauge. This filter, also called reflux filter herein-after, may advantageosly either be integrated into the enteral feeding catheter or into the device that houses the pressure gauge. If the reflux filter is integrated into the enteral feeding catheter, the filter is preferably integrated in the connector for connecting the measurement lumen to the device that houses the pressure gauge. However, the reflux filter may also advantageously cover the opening or openings of the measurement lumen towards the inside of the distal balloon.

The reflux filter is gas permeable in order to allow gas communication between the distal balloon and the pressure gauge. Further, the reflux filter delivers sufficient protection against passage of liquids.

Advantageously, the reflux filter may comprise a suitable gas-permeable membrane and a suitable, preferably hydrophobic, membrane. Various types of membranes can be suitable, such as microporous membranes, expanded PTFE membranes, membranes with urethane-coating to increase hydrophobic properties etc. Further, a different kind of porous body may be used, in particular if it is integrated in the device housing the pressure gauge or in the connector connecting the same to the measurement lumen of the enteral feeding catheter.

In case the reflux filter is integrated into the device that houses the pressure gauge, it is highly preferred to design the reflux filter exchangeable, e.g. as an exchangeable cartridge.

Preferably there are means to alert the medical staff in charge, if the reflux filter is full, which also indicates rupture or leakage of the distal balloon. Such means may be implemented in various ways, e.g. by measuring the resistance between two spaced apart electrodes disposed on the surface of a filter membrane or by providing a capacitive sensor.

The high degree of gastightness, which the distal balloon preferably has, allows relatively long recalibration intervals.

The inflatable and deflatable proximal balloon, which is preferably provided in addition to the distal balloon, is positioned in the patient's thorax when the catheter is inserted. For this, the distance between the proximal balloon and the distal balloon ranges advantageously between 10 and 70 cm, preferably between 15 and 30 cm, depending on the patient and the type of application. The proximal balloon is named "proximal balloon" just for the sake to give it a name and to distinguish it from the distal balloon which is situated distally with respect to the proximal balloon. "Proximal" must not be understood in the sense that the balloon is situated close to the proximal end of the catheter body. Providing the catheter with a proximal balloon which is situated in the thorax region of the patient, i.e. above the diaphragma, when the catheter is properly inserted in place, allows monitoring, in addition to monitoring the intra-abdominal pressure using the distal balloon, the intra-thoracic pressure, which often has a significant importance in monitoring cardiovascular parameters of a patient.

The blocking balloon, which is also preferably provided in addition to the distal balloon, is preferably filled with air or water, and can be very useful in assisting to place the catheter precisely and correctly without applying x-ray imaging. The catheter is advanced through the patient's esophagus with the blocking balloon being empty until the operator can be sure that the blocking balloon has entered the stomach. The balloon is then filled with air or water and the catheter is withdrawn until the blocking balloon abuts the cardia of patient's stomach, thus ensuring that the catheter is placed precisely with respect to the stomach. In addition, the blocking balloon prevents the catheter from being withdrawn further from its place. In order to avoid accidential overpressure, the pressure inside the balloon can preferably be limited and/or monitored. An overpressure would indicate falsely positioning of the blocking balloon in the esophagus.

Preferably, the blocking balloon is controlled manually using a counter balloon which is provided in fluid communication with the blocking balloon. For controlling the blocking balloon, a releasable check valve may also be included. (As a side note it is to be mentioned, that a counter balloon, with or without a releasable check valve, can also be advantageous in connection with operating blocking balloons of enteral feeding catheters which are not entirely designed according to the present invention.)

Including radiopaque material in the material of one or more of the balloons can significantly improve the control of positioning and inflating the balloons using X-ray-imaging. (As a side note it is to be mentioned, that radiopaque balloons can also be advantageous in connection with other applications of inflatable balloons in the medical field.)

Alternatively or additionally the feeding catheter may be provided with several radiopaque marking rings distributed over its length and/or nearby the balloons and/or a radiopaque stripe extending axially over its length to allow observing the placement of the catheter under X-ray-imaging. Also alternatively or additionally, visible insertion marks distributed over the length of the catheter may inform the operator on how far the catheter has been advanced into the patient's body.

Additionally, the feeding catheter may be provided with two or more electrodes placed near the distal end of the catheter for impedance measurement to determine the acidity of the gastric juice and to carry out gastric volume measurements. The gastric volume is negatively correlated with the bio-electrical impedance i.e. impedance decreases, if gastric volume increases. The gastric volume can be calculated with an empirically estimated function of the impedance.

Additionally or alternatively the feeding catheter may provide two or more electrodes sufficiently proximal before or after the proximal balloon. These electrodes should be placed above diaphragm in the esophagus in order to estimate transthoracic bioimpedance which measures cardiac stroke volume and cardiac output and thoracic fluid content. Additionally the feeding catheter may provide pulse densitometry means placed in the esophagus in order to measure arterial oxygen saturation or indo cyanine green dye concentration. It was found that this would be more reliable than common non invasive pulse densitometry means e.g. on the finger or ear.

For evaluation of the readings aquired by the various measurements performable using the enteral feeding catheter, suitable computing means may be used which are preferably integrated in the apparatus according to the present invention. Such computing means may provide for calibration and recalibration of the measurement equipment and allow continuous determination of the intra-abdominal pressure with good accuracy. They may be connected to pump driving means adapted to drive a pump for increasing/decreasing the gas volume inside at least one balloon of the enteral feeding catheter and to the pressure gauge. They include calculation means for calculating the mathematical derivative dp/dV of the pressure p with respect to volume V displaced by the pump and accessing means to access executable instructions to cause the computing means to cause the pump driving means to adjust the gas volume inside the distal balloon for initial setting such that said mathematical derivative dp/dV of pressure p with respect to volume V is zero or as close to zero as possible. Preferably the computing means are connected to valve means adapted to connect the pressure gauge to ambient air for initial settings and disconnect it from ambient air, wherein the computing means control connection of said pressure gauge to ambient air and to adjust said pressure gauge to zero. Advantageously the computing means further control alarm means alerting of a blocked or occluded catheter lumen, in case that the absolute mathematical derivative dp/dV of pressure p with respect to volume exceeds a certain upper threshold at a certain pressure. The computing means may further control alarm means alerting of a leakage in case that the absolute mathematical derivative dp/dV of pressure p with respect to volume V fails to reach a lower threshold at a certain pressure.

Generally, any of the embodiments described or options mentioned herein may be particularly advantageous depending on the actual conditions of application. Further, features of one embodiment may be combined with features of another embodiment as well as features known per se from the prior art as far as technically possible and unless indicated otherwise.

The invention will now be described in more detail. The accompanying drawings, which are exemplary schematic illustrations and not drawn to scale, serve for a better understanding of the features of the present invention and preferred embodiments thereof. Therein
- Fig. 1: is an overall view of a preferred embodiment of a feeding catheter according to the present invention;
- Fig. 2: is a longitudinal sectional view of the proximal part of the catheter of Fig. 1;
- Fig. 3: is a longitudinal sectional view of the middle part of the catheter of Fig. 1 taken along line III-III of Fig. 5;
- Fig. 4: is a longitudinal sectional view of the distal part of the catheter of Fig. 1 taken along line IV-IV of Fig. 6;
- Fig. 5: is a cross sectional view of the catheter taken along line V-V in Fig. 3;
- Fig.6: is a cross sectional view of the catheter taken along line VI-VI in Fig. 6;
- Fig. 7: is a cross sectional view of the catheter taken along line VII-VII in Fig. 4;
- Fig. 8: is a cross sectional view of the catheter taken along line VIII in Fig. 2;
- Fig. 9: is a diagrammatic view of part of the apparatus according to one aspect of the present invention;
- Fig. 10: is a diagram showing the pressure P measured with a pressure gauge as a function of the displacement x of a piston of a pump being part of the apparatus according to one aspect of the present invention;
- Fig. 11: is an overall view of a further embodiment of the feeding catheter according to the present invention equipped with a counter balloon and a check valve for controlling the blocking balloon.
- Fig. 12: is a longitudinal sectional view of a check valve as included in the embodiment of fig. 11.
- Fig. 13: is a longitudinal sectional view of a connector for connecting an enteral feeding catheter according to the present invention to a device housing a pressure gauge for measuring intra-abdominal pressure.
- Fig. 14a-d: are cross sectional views of various alternative ways of arranging a feeding lumen and a measurement lumen relativ to each other.

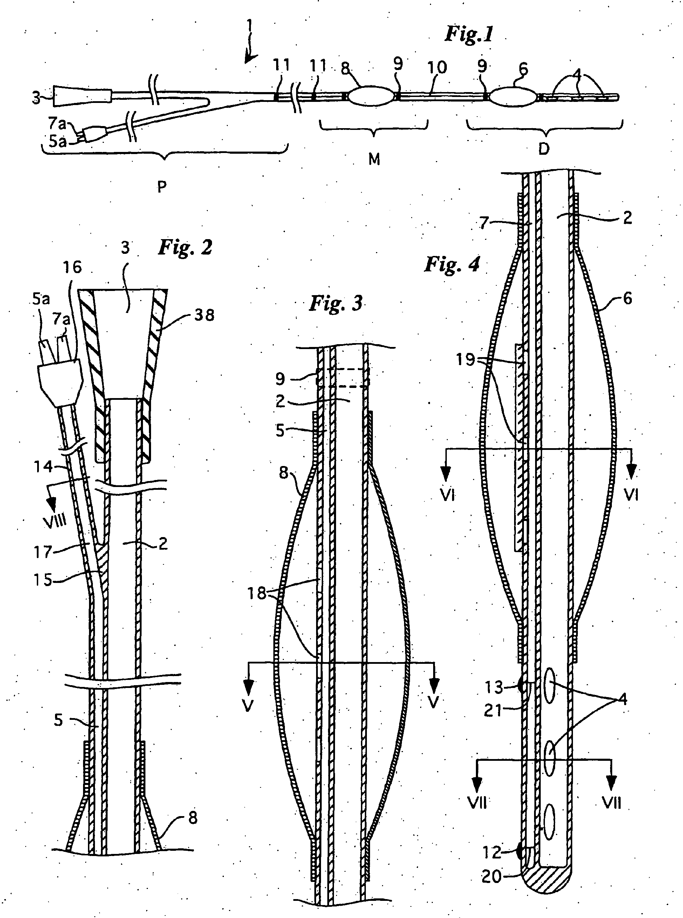

Fig. 1 shows a preferred embodiment of a feeding catheter according to the invention. The catheter 1 has three sections, namely a proximal section P, a middle section M and a distal section D. The catheter 1 has a proximal port with an opening 3 of the feeding lumen 2 (in fig. 1 not visible) by means of which it may be connected to a supply of nutrient, which may be hanged at a infusion stand (not shown) or which may include a food pump. There are two further openings, namely openings 5a and 7a in another proximal port, to which sources/sinks of gas pressure may be connected.

In the middle part M the body of the catheter 1 is surrounded by a proximal balloon 8 which is internally connected to the opening 5a, so that the proximal balloon 8 may be inflated by pressing air into the opening 5a or may be deflated by sucking air out of opening 5a which will be explained in detail later. The balloon 8 is named "proximal balloon" just for the sake to give it a name and to distinguish it from a balloon which is situated distally with respect to the proximal balloon which will be described later. "Proximal" is not to be understood that the balloon is situated at the proximal end of the catheter body.

In the distal part D the body of the catheter 1 is surrounded by a distal balloon 6 which is internally connected to the opening 7a, so that the distal balloon 6 may be inflated by pressing air into the opening 7a or may be deflated by sucking air out of the opening 7a which will be explained in detail later. The balloon 6 is named "distal balloon" just for the sake to give it a name and to distinguish it from the proximal balloon 8 which is situated proximally with respect to the distal balloon 6 mentioned before. "Distal" must not be understood that the balloon is situated at the very distal end of the catheter body. The ballon 6 may provide a suitable coating to avoid sticking of stomach contents on the balloon.

Distally with respect to the distal balloon 6 there are provided radial openings 4 which serve as outlets to allow flowable nutrients and/or medication, which have been introduced into the catheter 1 through its opening 3, to exit into patient's digestive tract.

Proximally and distally of and close to the balloons the catheter 1 has radio opaque marking rings 9, further it has an axially extending radio opaque stripe 10 and optically visible insertion marks 11. All of the above support the operator in determining the position of the catheter in patient's body, especially during the procedure of advancing it through the esophagusand the digestive tract of the patient. The insertion marks 11 may extend from balloon 8 up to the Y-junction 15 at equal distances (e.g. 5 cm).

To further improve controlled positioning of the catheter 1, one or both balloons 6 and 8 may contain radiopaque material in their walls. Radiopaque balloon walls further make it possible to view the process of inflation by using X-ray viewing.

The details of the structure of catheter 1 will become more apparent from figure 2 which shows a longitudinal section of the proximal part of the catheter 1. The proximal opening 3 of the catheter 1 is provided with a conical connector 38 to which a supply line for nutrients may be connected. The supply line (not shown) will disconnect from the connector 38 automatically if an excessive pulling force is exerted thereby protecting the patient from being injured. Downstream or distally of the connector 38 there is a first lumen 2 (fedding lumen), which serves to guide flowable nutrients or medicine down into patient's digestive tract.

A side hose 14 is connected to the main part of the catheter 1 via a Y-junction 15. The distal end of the side hose 14 is closed by a connector 16, which has two openings 5a and 7a. The side hose 14 is connected to a second lumen 5 (additional measurement lumen) and a third lumen (measurement lumen) which is not visible in figure 2. Inside the side hose 14 there is provided a lumen 5b which connects the opening 5a to the second lumen 5. There is a further lumen 7b in the side hose 14, which connects the second opening 7a to the third lumen 7 which is however not visible in the sectional view of figure 2. Downstream of the Y-junction 15 the second lumen 5 is parallel to the larger first lumen 2.

However, generally in the body of the catheter 1 the various lumina 2, 5, 7 may be arranged in a different manner relativ to each other, which will be described later.

As may be best understood from figure 3, the lumen 5 is connected to the proximal balloon 8. In the region where the body of the catheter 1 is surrounded by the proximal balloon 8 the outer wall of the second lumen 5 has one or more radial openings 18 which allow for gas flow between the second lumen 5 and the proximal balloon 8. As a result the interior of the proximal balloon 8 is connected to the opening 5a (figure 2) so that the proximal balloon 8 can be inflated by blowing air or another gas into the opening 5a. Both the proximal balloon 8 and the lumen 5 are optional as they are not used for measurement of the intra-abdominal pressure but the thoracic pressure of the patient.

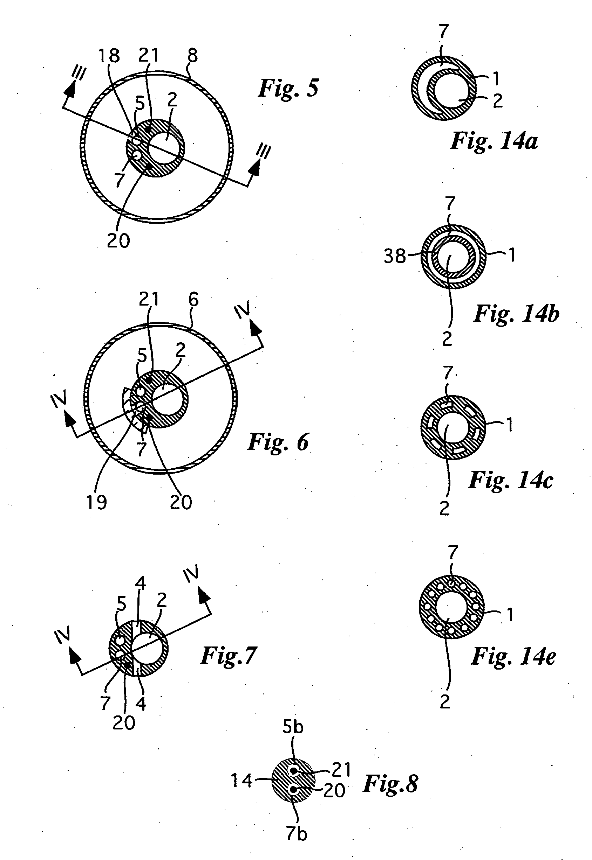

Figure 4 shows the distal part D of the catheter 1. It is to be obseved that - although the drawing of figure 4 looks similar to that of figure 3 - the plane of section is different to that of figure 3, which can be best understood from figures 5 and 6 in which the respective planes of section are indicated by arrows marked with "III" and "IV". As can be seen by comparing the respective figures, the plane of section for figure 3 traverses the second lumen 5 diametrally, while the plane of section of figure 4 traverses the third lumen 7 diametrally.

As becomes apparent from figure 4, the third lumen 7 is connected to the interior of the distal balloon 6 via radial openings 19. The interior of the distal balloon 6 communicates with the opening 7a and can hence be inflated and deflated through that opening 7a. The surface inside the balloons 6 and 8 is roughened or profiled in a way to ensure complete emptening of gas at deflation. The openings 19 are covered by a gas-permeable membrane 36 which functions as reflux filter and thus prevents, in case of rupture or leakage of the distal balloon 6, stomach juices from entering the third lumen 7.

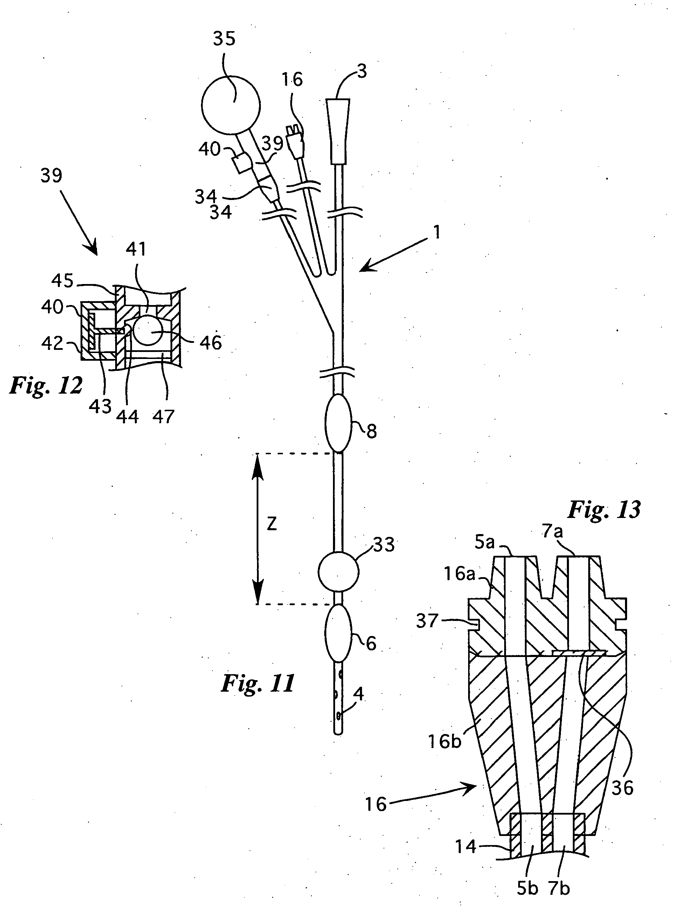

Alternatively, the reflux filter may be integrated into the connector 16 as illustrated in figure 13. The connector, in this exemplary embodiment, is manufactured from two pieces 16a, 16b, which may be mounted together by welding or use of adhesive. The lower connector piece 16b is fixed to side hose 14. The lumina 5b, 7b extend from the side hose 14 through both the lower and the upper connector pieces 16a, 16b to their respective openings 5a, 7a.

The upper connector piece 16a is designed to be connected to a a counterpart in order to connect the openings 5a, 7a of respective lumina 5b, 7b with a pressure gauge 24 and a gas pressure source/sink. This connection may be held together e.g. by a latch or snap fit mechanism. For this purpose the upper connector piece 16a comprises a circumferential groove 37 at its outside. Other types of connections, such as a bayonet coupling or a coupling nut are also possible alternatives.

In the upper connector piece 16a, as illustrated, or in the lower connector piece 16b a recess surrounding the lumen 7b (measurement lumen) is provided at the contact surface between the connector pieces 16a, 16b. The gas-permeable, microporous filter membrane 36 or porous filter body is disposed in the recess for preventing liquids in the lumen 7b from entering from the lower connector piece 16b into the upper connector piece 16a and thus from passing through the opening 7a.

Close to the distal tip of the catheter 1 the first lumen 2 is provided with several radial outlets 4 which allow nutrient to flow out of the first lumen 2. At the outer wall of the catheter 1 there are optionally provided two electrodes 12 and 13 spaced apart from each other. The electrodes 12, 13 are connected by electric wires 20 and 21 to the connector 16 (figure 2).

Figure 5 is a cross sectional view of the catheter taken along line V-V in figure 3. Figure 5 shows the catheter with its first lumen 2, second lumen 5, third lumen 7 and embedded connection wires 20, 21 for the electrodes 12, 13 shown in figure 4. Alternatively the wires 20 and 21 could be placed inside the lumina 5 and 7. The second lumen 5 is connected via openings 18 to the interior of the proximal balloon 8, while there is no such connection between the third lumen 7 and proximal balloon 8.

Figure 6 is a cross sectional view of the catheter taken along line IV-IV in figure 4. Figure 6 shows the catheter with its first lumen 2, second lumen 5, third lumen 7 and connection wires 20, 21 for the electrodes 12, 13 shown in figure 4. The third lumen 7 is connected via openings 19 to the interior of the distal balloon 6, while there is no such connection between the second lumen 5 and the distal balloon 6.

Figures 14a-d show various alternative ways of arranging a feeding lumen 2 and a lumen 7 for inflating/deflating a balloon in the body of a catheter 1. (As a side note it is to be mentioned, that any of these arrangements can also be advantageous in connection with other applications of catheters with inflateable/deflateable balloons in the medical field.) In Fig. 14a, the cross sectional area of the measurement lumen 7 is maximized due to crescent shape thereof thus reducing pressure drop and increasing inflation/deflation time. In Fig. 14b the same effect is achieved by coaxially arranging an inner hose 37 in the body of the catheter 1 to separate the outer (measurement) lumen 7 from the (inner) feeding lumen 2. The inner hose 37 may be arranged such that it can move laterally relative to the body of the catheter 1. The arrangements in figures 14c and 14b may also be called coaxial; however, the outer (measurement) lumen 7 is devided into several "sub-lumina" which are interconnected at at least one proximal and one distal location, e.g. in a connector at a proximal port of the catheter 1 and at the balloon to be inflated/deflated. The interconnection at the balloon to be inflated/deflated may be achieved simply by all sub-lumina opening into the balloon.

Figure 7 is a cross sectional view of the catheter taken along line VII-VII in figure 4. Figure 7 shows the catheter with its first lumen 2, second lumen 5, third lumen 7 and connection wire 20 for the electrode 12 shown in figure 4. The first lumen 2 has openings 4 allowing nutrient to flow out into patient's digestive tract.

Figure 8 is a cross sectional view of the catheter taken along line VIII in figure 2. Figure 2 shows the side hose 14 which is connected to the main part of the catheter 1 via a Y-junction 15 (figure 2). Inside the side hose 14 there are is provided the lumen 5b which connects the opening 5a to the second lumen 5 (figure 2). There is a further lumen 7b in the side hose 14, which connects the second opening 7a (figure 2) to the third lumen 7 (figure 4).

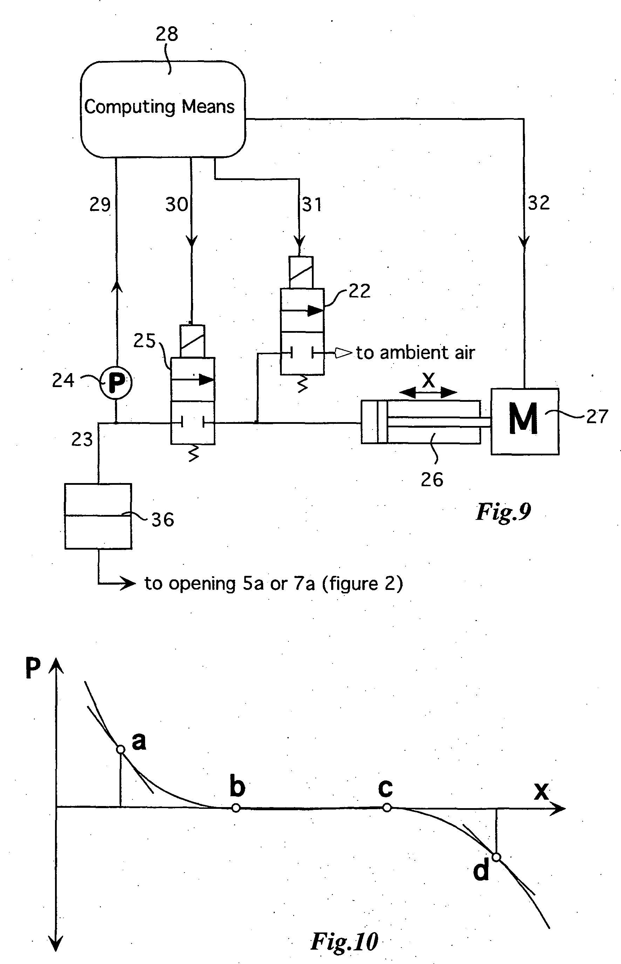

Figure 9 is a diagrammatic view of part of an apparatus according to the present invention or of a computerized system for operating the catheter 1 of the invention, respectively.

The catheter 1 is connected via its opening 7a to air conduit (or gas conduit) 23. The air conduit 23 may comprise a reflux filter element 36, preferably designed as an exchangeable filter cartridge. If a reflux filter element 36 is provided in the air conduit, a reflux filter 36 in the connector 16 or at the openings 19 of the catheter 1 are dispensable.

The air conduit 23 is connected to a pressure gauge 24, by which the pressure in the air conduit 23 can be measured continuously. The air conduit 23 is further connected over a first electromagnetic valve 25 to a piston pump 26 driven by a motor 27. The air conduit 23 is further connected to a second electromagnetic valve 22, by which the conduit may be connected to ambient air. A computer 28 is electrically connected via line 29 to the pressure gauge 24, via line 30 to the first electromagnetic valve 25, via line 31 to the second electromagnetic valve 22 and via line 32 to the electric motor 27. By means of these connections the computer 28 is able to collect pressure readings from the pressure gauge 24, to open and close the first and second valves 25 and 22 and to activate the motor 27 in order to advance or retract the piston of the piston pump 26. The catheter 1 may be connected via its opening 5a to an identical system or a switch valve (not shown) may be provided to connect the system shown in figure 9 selectively to the opening 7a and 5a of the catheter 1.

### The catheter system is operated as follows:

The catheter 1 is advanced with its distal tip ahead through the mouth or the nose of a patient into the esophagus of the patient until its tip reaches the stomach, the duodenum or the jejunum of the patient depending on the medical requirements. The axial position of the distal balloon 6 is such that the distal balloon will be placed in the stomach (or duodenum or jejunum), while the axial position of the proximal balloon 8 on the body of the catheter 1 is such that it will be placed in the patient's thorax. The movement of the catheter 1 is monitored by means of the radioopaque marking rings 9, radioopaque stripe 10 and/or by counting visible insertion marks 11 outside the patient.

Once the distal tip of the catheter 1 has reached its final destination in the patient's digestive tract, the computerized system as shown in figure 9 is connected to the opening 7a. The computer program that runs on the computer 28 causes the first and the second electromagnetic valves 22 and 25 to open, so that the air conduit 23 is connected to ambient air. Then the piston 26 is moved to a defined e.g. middle position and pressure gauge 24 is set to zero. The computer 28 then causes the electromagnetic valve 22 to close in order to disconnect the air conduit 23 from ambient air. The computer causes the motor 27 to drive the piston of piston pump 26 in forward direction so that air is blown into the the proximal balloon 8 via air conduit 23, opening 5a, lumen 7b, lumen 7 and openings 18, while taking pressure readings from the pressure gauge 24 and calculating the mathematical derivative dp/dV of pressure p with respect to volume V permanently. Instead of calculating dp/dV equally dp/dx may be calculated, where x is the position of the piston of the piston pump 26. The curves dp/dV and dp/dx have the same characteristic form and differ only by a constant scaling factor which corresponds to the (constant) cross section of the piston. The same applies for variables x and V, so that it does not make any difference if V or x is recorded.

During the inflation process the pressure inside the distal balloon 6 increases and the balloon 6 unfolds. After the balloon 6 has been unfolded the pressure inside the balloon increases rapidly, in other words the absolute amount of mathematical derivative of pressure p with respect to displacement x of the piston is high and exceeds a predetermined threshold at a certain positive pressure, causing the program to stop the inflation process and to start a deflation, in other words the piston will be moved in the opposite direction whereby the balloon 6 is evacuated. At the beginning of the deflation process the pressure falls rapidly until the balloon 6 begins to collapse. During the process of collapsing the balloon 6 the pressure changes only slightly or remains constant, in other words dp/dV and equally dp/dx are zero or at least very close to zero. When the balloon 6 has reached the state of being completely collapsed further movement of the piston will cause rapid pressure drop, in other words dp/dV and dp/dx respectively will have large absolute amounts. When the absolute amount of the derivative with respect to volume exceeds a predetermined threshold at a certain negative pressure the evacuation action will be stopped by the computer 28.

The diagram shown in figure 10 reflects the evacuation process. At the beginning (point a) the pressure drops rapidly and remains approximately constant between points b and c. Further evacuation, i.e. further withdrawal of the piston of the piston pump 26 causes an increasingly rapid pressure drop until point d is reached.

The pressure inside the stomach can however be assessed reliably when it is independent of the filling state of balloon 6, i.e. in the range between point b and point c.

After having collected the pressure readings and having calculated the derivative dp/dx (or dp/dV) the computer determines a position between points b and d, preferably the middle between points b and d, and moves the piston to this position to finalize the initial setting of the system.

In order to minimize leakage, valve 25 is closed after this calibration phase. If leakage is negligible valve 25 could be ommitted. Then the patient's intra-abdominal pressure can be assessed continuously. After some time due to inavoidable leakage e.g. of the balloon a recalibration may become indicated.

Assumed now that the balloon 6 or any other part as for example the connector 16 has a leakage. In this case the pressure p and its mathematical derivative dp/dV with respect to volume V will remain low during the process of inflation and the pressure and its derivative will remain below a predetermined threshold. This will be recognized by the computing means 28 and an alarm will be given.

Further it may occur that a lumen 5, 7 may be occluded or intentionally blocked because the catheter used has only one balloon. This situation as well will be recognized by the computing means 28, because in this case the pressure during the inflation process will go high very rapidly.

The initial adjustment for the proximal balloon 8 is done in the same way by connecting the opening 5a to the computerized system and proceeding in the same way as described above with respect to the distal balloon 6 in order to determine the patient's thoracic pressure. The computer may then calculate also the difference between abdominal and thoracal pressure and monitor this difference value.

While an embodiment has been described in which a catheter 1 with two balloons 6, 8 is employed, it goes without saying that only one balloon may be employed if the measurement of the pressure at only one site in patient's body is regarded to be sufficient. One of the two balloons 6, 8 and the respective catheter lumen 7, 5 pertaining to it may be left away.

Figure 11 is an overall view of a further embodiment of the feeding catheter according to the invention. The catheter has a proximal balloon 8 and a distal balloon 6 and is constructed mostly in the same way as the catheter described with reference to the drawings 1 to 8. However, the catheter is provided with an additional balloon 33 which serves as a blocking balloon 33. The balloon 33 is connected to an additional lumen (not shown in the drawing) in the same way as described with regard to balloons 6 and 8 of the first embodiment. In the embodiment of figure 11 the additional connector 34 is provided with one further opening allowing to supply a liquid under pressure into the balloon 33 and to withdraw it from the balloon 33.

To avoid syringe use, the blocking balloon 33 is manually controlled using a resilient counter balloon 35 the interior of which is in fluid communication with the blocking balloon 33. By squeezing the counter balloon 35, fluid is pushed from the counter balloon 35 into the blocking balloon 33 causing it to expand. If the wall of the counter balloon 35 is made of a material with sufficient stiffness, such that the counter balloon 35 seeks to regain its original shape, it will tend to suck the fluid back into the counter balloon 35 whenever the squeezing action stops. In order to keep the blocking balloon 33 expanded, a check valve 39 is provided between the additional connector 34 and the counter balloon 35 (but may also be integrated, for example, in the additional connector 34). By pressing on the operation element 40, the check valve 39 is released, and the counter balloon 35 can regain its original shape sucking back the fluid from the blocking balloon 33 causing the latter to collapse thus enabling the catheter 1 to be removed from its application site.

An example of the construction of a suitable check valve 39 is illustrated in Fig. 12 by way of a longitudinal sectional view. The check valve 39 comprises a valve seat 41 and a ball 46. If the counter balloon 35 is unfolding and sucking fluid back into its interior, the fluid is flowing upward in Fig. 12. As, when the the counter balloon 35 unfolds, the fluid pressure above the ball 46 is smaller than the fluid pressure below, the ball 46 is pressed into the valve seat 41 and thus stops the upword flow of the fluid (valve 39 in "sealed" condition). In order to release the check valve 39, the operation element 40 is to be pushed. The operation element 40 comprises a resilient cover 42 and a core 43. If the cover 42 is pushed, the core 43 will transmit the applied force via the knob 44 of the resilient wall 45 to the ball 46 causing the ball 46 to be moved sideways out of the valve seat 41 allowing fluid to flow upward through the valve seat 41.

If fluid is moved downward through the check valve 39 by squeezing the counter balloon 35 in order to expand the blocking balloon 33, the check valve 39 will automatically open, because the fluid pressure above the ball 46 is greater than the fluid pressure below. To keep the ball 46 from falling out of the check valve 39, a small cross bar 47 is provided.

The catheter of figure 11 is applied as follows:

The catheter 1 is advanced with its distal tip ahead through the mouth or the nose of a patient into the esophagus of the patient until its tip reaches the stomach, the duodenum or the jejunum of the patient depending on the medical requirements. The axial position of the distal balloon 6 is such that the distal balloon 6 as well as the blocking balloon 33 will then be situated in the stomach. The movement of the catheter 1 is monitored by means of counting the visible insertion marks 11 to make sure that the blocking balloon has entered the stomach. Then the blocking balloon 33 is filled with water and the catheter will be retracted until the blocking balloon 33 abuts the cardia of the patient's stomach, thus ensuring that the catheter is placed precisely with respect to the stomach and balloon 8 will be situated in the patient's thorax. Additionally the blocking balloon 33 prevents the catheter 1 from being withdrawn from its place. This blocking balloon 33 is very useful to assist in placing the catheter precisely and correctly without applying X-ray imaging.

The further operation of the catheter 1 is same as described for the catheter of the first embodiment and may be omitted therefore.

## Claims

1. Enteral feeding catheter (1) for channelling flowable nutrient into a patient's digestive tract, such as into patient's stomach, duodenum or jejunum,
said catheter (1) being adapted to be advanced through patient's esophagus,
said catheter (1) having a distal end (D) and a feeding lumen (2) with a proximal end (P), said feeding lumen (2) having an opening (3) at its proximal end (P) adapted to be connected to a supply for receiving said nutrient from said supply and channelling said nutrient from said proximal end (P) to said distal end (D),
said distal end (D) of said feeding lumen (2) being provided with at least one outlet (4) for the exit of said nutrient into patient's digestive tract,
said catheter (1) further comprising a measurement lumen (7) connected to an inflatable and deflatable distal balloon (6) provided on said catheter (1) near said distal end (D) and a proximal end (7a), and
connecting means (16) to connect said proximal end (7a) of said measurement lumen (7) with a source/sink of gas (26) and a gas pressure gauge (24) at said proximal end (7a) of said measurement lumen (7), **characterized in that,**
a reflux filter (36) for protection against passage of liquids is arranged between said distal balloon (6) and said proximal end (7a) of said measurement lumen (7), and
said reflux filter (36) being gas-permeable in order to provide gas communication between the distal balloon (6) and said proximal end (7a).

2. Enteral feeding catheter according to claim 1, wherein said reflux filter (36) comprises a gas-permeable membrane.

3. Enteral feeding catheter according to claim 2, wherein said membrane is hydrophobic.

4. Enteral feeding catheter according to any of the proceeding claims, further comprising an additional measurement lumen (5) having a proximal end (5a) adapted to be connected to a source/sink of gas (26) and a gas pressure gauge (24), said additional measurement lumen (5) being connected to an inflatable and deflatable proximal balloon (8) provided on said catheter (1) at an axial position such that said proximal balloon (8) is positioned in the patient's thorax when the catheter (1) is inserted and the distal balloon (6) is positioned in the patient's stomach.

5. Enteral feeding catheter according to claim 4, wherein the axial distance (Z) between said distal balloon (6) and said proximal balloon (8) is between 10 cm and 70 cm.

6. Enteral feeding catheter according to claim 5, wherein the axial distance (Z) between said distal balloon (6) and said proximal balloon (8) is between 15 cm and 30 cm.

7. Enteral feeding catheter according to any of the preceding claims, further comprising a blocking-control lumen having a proximal end adapted to be connected to a source/sink of fluid at the proximal end of said blocking-control lumen and being connected to a blocking balloon (33) provided on said catheter at an axial position such that said blocking balloon (33) is positioned in patient's stomach in a position proximal relative to said distal balloon (6) when the catheter is inserted.

8. Enteral feeding catheter according to claim 7, wherein said proximal end of said blocking-control lumen is connected to said source/sink of fluid, and said source/sink of fluid comprises a manually operable counter balloon (35).

9. Enteral feeding catheter according to any of claims 7-8, wherein a releasable check valve (39) is arranged between said proximal end of said blocking-control lumen and said source/sink of fluid.

10. Enteral feeding catheter (1) according to any of the preceding claims, further comprising a plurality radiopaque marking rings (9) distributed over its length.

11. Enteral feeding catheter (1) according to any of the preceding claims, further comprising a radiopaque marking (11) extended over its length.

12. Enteral feeding catheter (1) according to any of the preceding claims, wherein at least one balloon (6, 8, 33) is radiopaque.

13. Enteral feeding catheter (1) according to any of the preceding claims, further comprising several insertion marks (10) distributed over its length.

14. Enteral feeding catheter (1) according to any of the preceding claims, further comprising at least two electrodes (12, 13) placed near the distal end (D) of the catheter (1) for impedance measurement and gastric volume measurement.

15. Apparatus for determining the intra-abdominal pressure of a patient, said apparatus comprising
a source/sink of gas (26),
a gas pressure gauge (24), and
an enteral feeding catheter (1) according to claim 1,
wherein said reflux filter is arranged between said distal ballon (6) and said pressure gauge (24),
wherein said pressure gauge (24) is adapted to determine the pressure p prevailing in said distal balloon (6).

16. Apparatus according to claim 15, wherein said catheter is a catheter according to any of claims 1-14.

17. Apparatus according to claim 15, wherein said reflux filter (36) is arranged between said proximal end (7a) of said measurement lumen (7) and said pressure gauge.

18. Apparatus according to claim 17, wherein said reflux filter (36) is exchangeable.

19. Apparatus according to claim 18, wherein said reflux filter (36) is integrated in an exchangeable cartridge.

20. Apparatus according to any of claims 15-19, wherein said apparatus comprises means for determining whether said reflux filter is charged by substances originating from the patient's stomach.

21. Apparatus according to any of claims 15-20 wherein said source/sink of gas (26) comprises a pump (26) for increasing/decreasing the gas volume V inside said distal balloon (6) of said enteral feeding catheter, and said apparatus calculation means for calculating the mathematical derivative dp/dV of said pressure p with respect to said gas volume V and computing means adapted to cause said pump (26) to adjust the gas volume inside said distal balloon (6) for an initial setting such that said mathematical derivative dp/dV of said pressure p with respect to said volume V is zero or as close to zero as possible.

22. Apparatus according to claim 21 further comprising valve means (22, 25) adapted to be set to connect said pressure gauge (24) to ambient air for initial settings and and to be set to disconnect said pressure gauge (24) from ambient air.

23. Apparatus according to claim 21 or 22 further comprising alarm means alerting of a blocked or occluded measurement lumen 7, in case that the mathematical derivative dp/dV of pressure p with respect to volume V exceeds a predetermined upper threshold at a predetermined pressure.

24. Apparatus according to any of claims 21-23, further comprising alarm means alerting of a leakage in case that the mathematical derivative dp/dV of pressure p with respect to volume V fails to reach a lower threshold at a certain pressure.

## Patentansprüche

1. Katheter (1) zur enteralen Ernährung zum Leiten einer fließfähigen Nährstofflösung in den Verdauungstrakt eines Patienten, wie zum Beispiel in den Magen, das Duodenum oder das Jejunum des Patienten,
wobei der Katheter (1) dazu ausgelegt ist, durch den Ösophagus des Patienten eingeschoben zu werden,
wobei der Katheter (1) ein distales Ende (D) und ein Ernährungslumen (2) mit einem proximalen Ende (P) hat,
wobei das Ernährungslumen (2) eine Öffnung (3) an seinem proximalen Ende (P) hat, das dazu ausgelegt ist, an eine Versorgung angeschlossen zu werden, um die Nährstofflösung von der Versorgung zu empfangen und um die Nährstofflösung von dem proximalen Ende (P) zu dem distalen Ende (D) zu leiten,
wobei das distale Ende (D) des Ernährungslumens (2) mit mindestens einem Auslass (4) zum Entlassen der Nährstofflösung in den Verdauungstrakt des Patienten versehen ist,
wobei der Katheter (1) ferner ein Messlumen (7), das mit einem aufpumpbaren und auslassbaren Ballon (6) verbunden ist, der in der Nähe des distalen Endes (D) auf dem Katheter (1) vorgesehen ist, sowie ein proximales Ende (7a) umfasst, und
ein Verbindungsmittel (16) zum Verbinden des proximalen Endes (7a) des Messlumens (7) mit einer Quelle/Senke von Gas (26) und einem Gasdruckmesser (24) an dem proximalen Ende (7a) des Messlumens (7), **dadurch gekennzeichnet, dass**
ein Rückflussfilter (36) zum Schutz gegen das Hindurchgelangen von Flüssigkeiten zwischen dem distalen Ballon (6) und dem proximalen Ende (7a) des Messlumens (7) vorgesehen ist, und
der Rückflussfilter (36) gasdurchlässig ist, um eine Gaskommunikation zwischen dem distalen Ballon (6) und dem proximalen Ende (7a) vorzusehen.

2. Katheter (1) zur enteralen Ernährung nach Anspruch 1, wobei der Rückflussfilter (36) eine für Gas durchlässige Membran umfasst.

3. Katheter (1) zur enteralen Ernährung nach Anspruch 2, wobei die Membran hydrophob ist.

4. Katheter (1) zur enteralen Ernährung nach einem der vorhergehenden Ansprüche, ferner umfassend ein zusätzliches Messlumen (5), das ein proximales Ende (5a) hat, das dazu ausgelegt ist, mit einer Quelle/Senke von Gas (26) und einem Gasdruckmesser (24) verbunden zu werden, wobei das zusätzliche Messlumen (5) mit einem aufpumpbaren und auslassbaren proximalen Ballon (8) verbunden ist, der auf dem Katheter (1) an einer axialen Position vorgesehen ist, so dass der proximale Ballon (8) in dem Thorax des Patienten angeordnet ist, wenn der Katheter (1) eingeführt ist, und der distale Ballon (6) in dem Magen des Patienten angeordnet ist.

5. Katheter (1) zur enteralen Ernährung nach Anspruch 4, wobei der axiale Abstand (Z) zwischen dem distalen Ballon (6) und dem proximalen Ballon (8) zwischen 10 cm und 70 cm beträgt.

6. Katheter (1) zur enteralen Ernährung nach Anspruch 5, wobei der axiale Abstand (Z) zwischen dem distalen Ballon (6) und dem proximalen Ballon (8) zwischen 15 cm und 30 cm beträgt.

7. Katheter (1) zur enteralen Ernährung nach einem der vorhergehenden Ansprüche, ferner umfassend ein Blockierungskontrolllumen, das ein proximales Ende hat, das dazu ausgelegt ist, mit einer Quelle/Senke von Fluid an dem proximalen Ende des Blockierungskontrolllumens verbunden zu werden und mit einem Blockierballon (33) verbunden ist, der auf dem Katheter an einer axialen Position vorgesehen ist, so dass der Blockierballon (33) in dem Magen des Patienten in einer Position proximal relativ zu dem distalen Ballon (6) angeordnet ist, wenn der Katheter eingeführt ist.

8. Katheter (1) zur enteralen Ernährung nach Anspruch 7, wobei das proximale Ende des Blockierungskontolllumens mit einer Quelle/Senke von Fluid verbunden ist und die Quelle/Senke von Fluid einen manuell zu betätigenden Gegenballon (35) umfasst.

9. Katheter (1) zur enteralen Ernährung nach einem der Ansprüche 7 bis 8, wobei ein lösbares Rückschlagventil (39) zwischen dem proximalen Ende des Blockierungskontrolllumens und der Quelle/Senke von Fluid angeordnet ist.

10. Katheter (1) zur enteralen Ernährung nach einem der vorhergehenden Ansprüche, ferner umfassend eine Vielzahl von stahlungsundurchlässigen Markierungsringen (9), die über seine Länge verteilt sind.

11. Katheter (1) zur enteralen Ernährung nach einem der vorhergehenden Ansprüche, ferner umfassend eine stahlungsundurchlässige Markierung (11), die sich über seine Länge erstreckt.

12. Katheter (1) zur enteralen Ernährung nach einem der vorhergehenden Ansprüche, wobei mindestens ein Ballon (6, 8, 33) stahlungsundurchlässig ist.

13. Katheter (1) zur enteralen Ernährung nach einem der vorhergehenden Ansprüche, ferner umfassend mehrere Einführungsmarken (10), die über seine Länge verteilt sind.

14. Katheter (1) zur enteralen Ernährung nach einem der vorhergehenden Ansprüche, ferner umfassend mindestens zwei Elektroden (12, 13), die zur Impedanzmessung und Messung des Magenvolumens in der Nähe des distalen Endes (D) des Katheters (1) angeordnet sind.

15. Vorrichtung zum Bestimmen des intraabdominalen Drucks eines Patienten, wobei die Vorrichtung umfasst:
eine Quelle/Senke von Gas (26),
einen Gasdruckmesser (24), und
einen Katheter (1) zur enteralen Ernährung nach Anspruch 1,
wobei der Rückflussfilter zwischen dem distalen Ballon (6) und dem Druckmesser (24) angeordnet ist,
wobei der Druckmesser (24) dazu ausgelegt ist, den in dem distalen Ballon (6) vorherrschenden Druck p zu bestimmen.

16. Vorrichtung nach Anspruch 15, wobei der Katheter ein Katheter nach einem der Ansprüche 1 bis 14 ist.

17. Vorrichtung nach Anspruch 15, wobei der Rückflussfilter (36) zwischen dem proximalen Ende (7a) des Messlumens (7) und dem Druckmesser angeordnet ist.

18. Vorrichtung nach Anspruch 17, wobei der Rückflussfilter (36) austauschbar ist.

19. Vorrichtung nach Anspruch 18, wobei der Rückflussfilter (36) in eine austauschbare Patrone integriert ist.

20. Vorrichtung nach einem der Ansprüche 15 bis 19, wobei die Vorrichtung ein Mittel zum Bestimmen umfasst, ob der Rückflussfilter mit Substanzen besetzt ist, die aus dem Magen des Patienten stammen.

21. Vorrichtung nach einem der Ansprüche 15 bis 20, wobei die Quelle/Senke von Gas (26) eine Pumpe (26) umfasst zum Erhöhen/Verringern des Gasvolumens V innerhalb des distalen Ballons (6) des Katheters zur enteralen Ernährung, und die Vorrichtung ein Berechnungsmittel zum Berechnen der mathematischen Ableitung dp/dV des Drucks p nach dem Gasvolumen V sowie ein Berechnungsmittel umfasst, das dazu ausgelegt ist, die Pumpe (26) zu veranlassen, das Gasvolumen innerhalb des distalen Ballons (6) für eine Anfangseinstellung so einzustellen, dass die mathematische Ableitung dp/dV des Drucks p nach dem Volumen V null ist oder null so nahe wie möglich ist.

22. Vorrichtung nach Anspruch 21, ferner umfassend ein Ventilmittel (22, 25), das dazu ausgelegt ist, so eingestellt zu werden, dass es für Anfangseinstellungen den Druckmesser (24) mit der Umgebungsluft verbindet, und so eingestellt zu werden, dass es den Druckmesser (24) von der Umgebungsluft trennt.

23. Vorrichtung nach Anspruch 21 oder 22, umfassend ein Alarmmittel, das ein verstopftes oder versperrtes Messlumen 7 anzeigt, falls die mathematische Ableitung dp/dV des Drucks p nach dem Volumen V bei einem vorbestimmten Druck einen vorbestimmten oberen Schwellenwert überschreitet.

24. Vorrichtung nach einem der Ansprüche 21 bis 23, ferner umfassend ein Alarmmittel, das eine Leckage anzeigt, falls die mathematische Ableitung dp/dV des Drucks p nach dem Volumen V bei einem vorbestimmten Druck einen unteren Schwellenwert nicht erreicht.

## Revendications

1. Sonde d'alimentation entérale (1) pour acheminer des nutriments fluides dans le tube digestif d'un patient, tel que l'estomac, le duodénum ou le jéjunum du patient,
ladite sonde (1) étant apte à être avancée à travers l'oesophage du patient,
ladite sonde (1) ayant une extrémité distale (D) et une lumière d'alimentation (2) avec une extrémité proximale (P),
ladite lumière d'alimentation (2) ayant une ouverture (3) à son extrémité proximale (P) apte à être reliée à une alimentation pour recevoir lesdits nutriments à partir de ladite alimentation et acheminer lesdits nutriments de ladite extrémité proximale (P) à ladite extrémité distale (D),
ladite extrémité distale (D) de ladite lumière d'alimentation (2) comportant au moins une sortie (4) pour la sortie desdits nutriments dans le tube digestif du patient,
ladite sonde (1) comprenant en outre une lumière de mesure (7) reliée à un ballonnet distal gonflable et dégonflable (6) disposé sur ladite sonde (1) à proximité de ladite extrémité distale (D) et d'une extrémité proximale (7a), et
des moyens de liaison (16) pour relier ladite extrémité proximale (7a) de ladite lumière de mesure (7) avec une source/puits de gaz (26) et un manomètre de gaz (24) à ladite extrémité proximale (7a) de ladite lumière de mesure (7), **caractérisée par le fait que**,
un filtre de reflux (36) pour une protection contre le passage de liquides est disposé entre ledit ballonnet distal (6) et ladite extrémité proximale (7a) de ladite lumière de mesure (7), et
ledit filtre de reflux (36) étant perméable aux gaz dans le but d'assurer une communication de gaz entre le ballonnet distal (6) et ladite extrémité proximale (7a).

2. Sonde d'alimentation entérale selon la revendication 1, dans laquelle ledit filtre de reflux (36) comprend une membrane perméable aux gaz.

3. Sonde d'alimentation entérale selon la revendication 2, dans laquelle ladite membrane est hydrophobe.

4. Sonde d'alimentation entérale selon l'une quelconque des revendications précédentes, comprenant en outre une lumière de mesure supplémentaire (5) ayant une extrémité proximale (5a) apte à être reliée à une source/puits de gaz (26) et un manomètre de gaz (24), ladite lumière de mesure supplémentaire (5) étant reliée à un ballonnet proximal gonflable et dégonflable (8) disposé sur ladite sonde (1) à une position axiale de telle sorte que ledit ballonnet proximal (8) est positionné dans le thorax du patient lorsque la sonde (1) est introduite et que le ballonnet distal (6) est positionné dans l'estomac du patient.

5. Sonde d'alimentation entérale selon la revendication 4, dans laquelle la distance axiale (Z) entre ledit ballonnet distal (6) et ledit ballonnet proximal (8) est entre 10 cm et 70 cm.

6. Sonde d'alimentation entérale selon la revendication 5, dans laquelle la distance axiale (Z) entre ledit ballonnet distal (6) et ledit ballonnet proximal (8) est entre 15 cm et 30 cm.

7. Sonde d'alimentation entérale selon l'une quelconque des revendications précédentes, comprenant en outre une lumière de commande de blocage ayant une extrémité proximale apte à être reliée à une source/puits de fluide à l'extrémité proximale de ladite lumière de commande de blocage et qui est reliée à un ballonnet de blocage (33) disposé sur ladite sonde à une position axiale de telle sorte que ledit ballonnet de blocage (33) est positionné dans l'estomac du patient dans une position proximale par rapport audit ballonnet distal (6) lorsque la sonde est introduite.

8. Sonde d'alimentation entérale selon la revendication 7, dans laquelle ladite extrémité proximale de ladite lumière de commande de blocage est reliée à ladite source/puits de fluide, et ladite source/puits de fluide comprend un contre-ballonnet (35) apte à être actionné manuellement.

9. Sonde d'alimentation entérale selon l'une quelconque des revendications 7-8, dans laquelle un clapet de non-retour déblocable (39) est disposé entre ladite extrémité proximale de ladite lumière de commande de blocage et ladite source/puits de fluide.

10. Sonde d'alimentation entérale (1) selon l'une quelconque des revendications précédentes, comprenant en outre une pluralité d'anneaux de marquage radio-opaque (9) répartis sur sa longueur.

11. Sonde d'alimentation entérale (1) selon l'une quelconque des revendications précédentes, comprenant en outre un marquage radio-opaque (11) s'étendant sur sa longueur.

12. Sonde d'alimentation entérale (1) selon l'une quelconque des revendications précédentes, dans laquelle au moins un ballonnet (6, 8, 33) est radio-opaque.

13. Sonde d'alimentation entérale (1) selon l'une quelconque des revendications précédentes, comprenant en outre plusieurs repères d'introduction (10) répartis sur sa longueur.

14. Sonde d'alimentation entérale (1) selon l'une quelconque des revendications précédentes, comprenant en outre au moins deux électrodes (12, 13) placées à proximité de l'extrémité distale (D) de la sonde (1) pour une mesure d'impédance et une mesure de volume gastrique.

15. Appareil pour déterminer la pression intra-abdominale d'un patient, ledit appareil comprenant
- une source/puits de gaz (26),
- un manomètre de gaz (24), et
- une sonde d'alimentation entérale (1) telle que définie à la revendication 1,
- ledit filtre de reflux étant disposé entre ledit ballonnet distal (6) et ledit manomètre (24),
- ledit manomètre (24) étant apte à déterminer la pression p régnant dans ledit ballonnet distal (6).

16. Appareil selon la revendication 15, dans lequel ladite sonde est une sonde telle que définie à l'une quelconque des revendications 1 - 14.

17. Appareil selon la revendication 15, dans lequel ledit filtre de reflux (36) est disposé entre ladite extrémité proximale (7a) de ladite lumière de mesure (7) et ledit manomètre.

18. Appareil selon la revendication 17, dans lequel ledit filtre de reflux (36) est échangeable.

19. Appareil selon la revendication 18, dans lequel ledit filtre de reflux (36) est intégré dans une cartouche échangeable.

20. Appareil selon l'une quelconque des revendications 15 - 19, ledit appareil comprenant des moyens pour déterminer si ledit filtre de reflux est chargé de substances provenant de l'estomac du patient.

21. Appareil selon l'une quelconque des revendications 15 - 20, dans lequel ladite source/puits de gaz (26) comprend une pompe (26) pour augmenter/diminuer le volume de gaz V à l'intérieur dudit ballonnet distal (6) de ladite sonde d'alimentation entérale, et ledit appareil comprend des moyens de calcul pour calculer la dérivée mathématique dp/dV de ladite pression p par rapport audit volume de gaz V et des moyens informatiques aptes à amener ladite pompe (26) à régler le volume de gaz à l'intérieur dudit ballonnet distal (6) pour un réglage initial tel que ladite dérivée mathématique dp/dV de ladite pression p par rapport audit volume V est zéro ou aussi proche de zéro que possible.

22. Appareil selon la revendication 21, comprenant en outre des moyens formant soupapes (22, 25) aptes à être réglés pour relier ledit manomètre (24) à l'air ambiant pour des réglages initiaux et à être réglés pour séparer ledit manomètre (24) de l'air ambiant.

23. Appareil selon la revendication 21 ou la revendication 22, comprenant en outre des moyens d'alarme pour une alerte d'une lumière de mesure bloquée ou occluse (7), dans le cas où la dérivée mathématique dp/dV de la pression p par rapport au volume V dépasse un seuil supérieur prédéterminé à une pression prédéterminée.

24. Appareil selon l'une quelconque des revendications 21 - 23, comprenant en outre des moyens d'alarme pour une alerte d'une fuite dans le cas où la dérivée mathématique dp/dV de la pression p par rapport au volume V n'atteint pas un seuil inférieur à une pression donnée.
